(19) Europäisches Patentamt
European Patent Office
Office européen des brevets

(11) Veröffentlichungsnummer : **0 406 572 B1**

(12) **EUROPÄISCHE PATENTSCHRIFT**

(45) Veröffentlichungstag der Patentschrift :
**28.04.93 Patentblatt 93/17**

(51) Int. Cl.$^5$ : **C07C 35/36,** C07C 29/58, C07C 35/52, C07C 29/132, A61K 7/46, C07D 303/04

(21) Anmeldenummer : **90110572.6**

(22) Anmeldetag : **05.06.90**

(54) **Cyclische Verbindungen und Verfahren zu deren Herstellung.**

(30) Priorität : **13.06.89 EP 89810451**

(43) Veröffentlichungstag der Anmeldung :
**09.01.91 Patentblatt 91/02**

(45) Bekanntmachung des Hinweises auf die Patenterteilung :
**28.04.93 Patentblatt 93/17**

(84) Benannte Vertragsstaaten :
**CH DE ES FR GB IT LI NL**

(56) Entgegenhaltungen :
**WO-A-82/00030**
**DE-A- 2 733 938**

(73) Patentinhaber : **L. GIVAUDAN & CIE Société Anonyme**
**CH-1214 Vernier-Genève (CH)**

(72) Erfinder : **Naegeli, Peter, Dr.**
**Vordere Höhenstrasse 31**
**CH-5430 Wettingen (CH)**

(74) Vertreter : **Urech, Peter, Dr. et al**
**Grenzacherstrasse 124 Postfach 3255**
**CH-4002 Basel (CH)**

**EP 0 406 572 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung der Verbindung der Formel

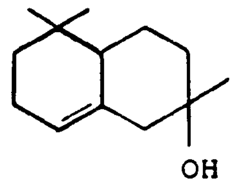

I

also von 2-Hydroxy-2,5,5-trimethyl-1,2,3,4,4a,5,6,7-octahydronaphthalin (α-Ambrinol).

Das Verfahren ist dadurch gekennzeichnet, dass man ein 8-Chlor oder Brom-2-hydroxy-2,5,5-trimethyl-2,3,4,4a,5,6,7,8-octahydronaphthalin, gegebenenfalls im Gemisch mit einem 1-Chlor oder Brom-2-hydroxy-2,5,5-trimethyl-1,2,3,4,4a,5,6,7, -octahydronaphthalin, bzw. das 1,2-Epoxyd davon, reduziert.

Das Verfahren kann wie folgt visualisiert werden:

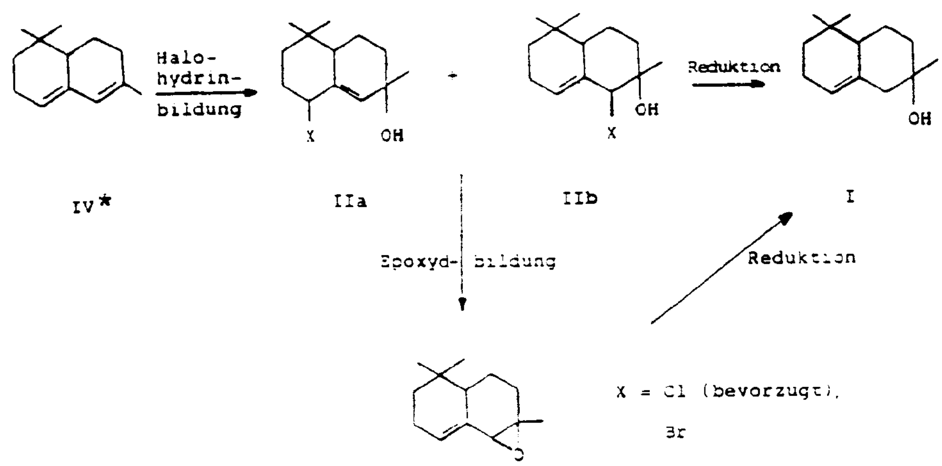


IV*, IIa, IIb, I, III

X = Cl (bevorzugt), Br

* G. Büchi et al., J. Am. Chem. Soc. 78, 2622 (1956)

Die Formeln I, II, III und IV sollen alle durch die asymmetrischen Zentren bedingten Stereoisomeren umfassen: Die Formel IV weist ein asymmetrisches Zentrum auf, die Formeln IIa, IIb, III deren drei und Formel I deren zwei. Im Falle von I soll also racemisches α-Ambrinol als auch racemisches epi α-Ambrinol sowie deren optisch aktive Enantiomeren umfasst werden.

Die geeigneten Verfahrensparameter sind die folgenden:

$$IV \xrightarrow{HOX} II$$

X = Cl, Br

| | |
|---|---|
| Methodik | nach den an sich bekannten Methoden der Halohydrinbildung aus ungesättigten Kohlenwasserstoffen, also mittels Hypohalogeniten, z.B. NaOX, $Ca(OX)_2$ (z.B. Javellelauge, Chlorkalk) etc. und sauren Verbindungen wie org. oder anorg. Säuren oder deren sauren Salzen |
| Medium | org./wässrige Phase [Aether wie THF, Dioxan, Glykoläther, Poly- äthylenglykoläther, tert. Alkohole, z.B. tert.Butanol]. |
| | pH 2-7, vorzugsweise ca. 5-6, vorzugsweise gepuffert |

Temperatur -10 bis +35°C, insbesondere ca. 5 bis ca. 15°C
Aufarbeitung übl. Aufarbeitung, z.B. Extraktion mit (chlorierten) Kohlenwasser- stoffen, Aethern. Estern, etc.
Trennung IIa/IIb unnötig, aber möglich, z.B. durch Chromatographie, z.B. Adsorptionschromatographie
Isomerenverhältnis: IIa>IIb

II → III

nach den an sich bekannten Methoden der Epoxydbildung aus Halohydrinen: geeignet sind also Alkaliamide wie Lithiumdiispropylamid, Lithiumdicyclohexylamid, Natriumamid, etc. Alkalihydride, Alkali-t-butanolate, Alkali-t-amylate, Dimethylsulfoxidanion, Alkalihydroxide/Phasentransfer
wasserfrei in Aethern, aliphat. oder aromatischen Kohlenwasserstoffen, tert. Alkoholen, etc.; oder zweiphasig mit Phasentransferkatalysatoren wie z.B. quat. Ammonium- und Phosphoniumverbindungen, Kronenäthern, Kryptaten oder Glykoläthern bzw. Aminoglykoläther
ca. -20° bis ca. 120°C, vorzugsweise ca. 10 bis ca. 80°C
Extraktion mit (halog.) Kohlenwasserstoffen, Aethern, etc.

II → I
III → I

nach den an sich bekannten Methoden der Reduktion von Halohydrinen bzw. von Allylhalogeniden bzw. der reduktiven Oeffnung von Epoxyden, geeignet sind also komplexe Hydride, z.B. $LiAlH_4$, $LiAl(OR)_xH_{4-x}$, $LiBR_3H$, $NaAlR_xH_{4-x}$, $NaAl(OR)_xH_{4-x}$, [R = nieder-Alkyl, insbes. $C_{1-6}$-Alkyl, bevorzugt Aethyl, x = 1,2,3], z.B. "Redal", "Vitride"
Wahl des Lösungsmittels und des Temperaturbereichs insbes. abhängig vom Reduktionsmittel, geeignet sind insbesondere ätherische Lösungsmittel oder Kohlenwasserstoffe
II → I
ca. -30°C bis ca. 100°C
insb. ca. 0°C bis ca. 80°C
III → I
ca. -30°C bis ca. 100°C
insb. ca. 0°C bis ca. 80°C
Hydrolyse des intermediär gebildeten Alkoholats, Extraktion mit (halog.) Kohlenwasserstoffen, Aethern, Estern, etc.
Isomerentrennung:
Adsorptionschromatographie, frakt. Destillation (Anreicherung des gewünschten Produkts)

Es sind zwar mehrere Synthesen zur Herstellung von Ambrinol bekannt, siehe z.B. Strickler, DT-OS 2733928 (a), wo von β-Jonon ausgegangen wird, oder Willis et al., US-PS 4341903 (b) wo von α-Jonon bzw. α-Dihydrojonon ausgegangen wird.

Die vorliegende Synthese ist technologisch einfach, sie vermeidet einerseits Hochtemperaturgasphasenreaktionen als auch aufwendige Fraktionierverfahren (für das unerwünschte Edukt) (→a) bzw. Festkörperchemie (Zn +$H_3PO_3$) (→b). Zudem ist das nach dem neuen Verfahren anfallende α-Ambrinol direkt für die Parfümerie verwendbar, die anfallenden Nebenprodukte stören organoleptisch nicht.

Im Verlaufe der vorliegenden Arbeiten wurde zudem gefunden, dass die neue Verbindung III über wertvolle Riechstoffeigenschaften verfügt und deshalb als Riechstoff verwendet werden kann.

Die Erfindung betrifft deshalb auch die Verwendung von III als Riechstoff.

Die Geruchsnoten von III können wie folgt charakterisiert werden: fäkalisch, animalisch, nach Schweiss, Urin von Wildkatzen.

Diese Noten sind überraschend, denn nahe verwandte Epoxyde, siehe z.B.

E.T. Theimer, Fragrance Chemistry, Acad. Press 1982, S. 552,553 und

G. Ohloff, Chemie in unserer Zeit 5, 114 (1971)

liessen einen Ambroxgeruch (=Ambroxan: warm, tabakartig) und Holzgeruch (Sandelholz) erwarten. Diese Noten sind jedoch abwesend. Auch die im Stand der Technik genannten Nebennoten - nämlich Eukalyptus, Kampfer, fehlen vollkommen.

Die Verbindung der Formel III eignet sich aufgrund ihrer Geruchsnoten insbesondere zur Modifizierung und Verstärkung von bekannten Kompositionen. Hervorgehoben werden soll insbesondere ihre ausserordentliche Geruchsstärke, die ganz allgemein zur Veredlung der Kompositionen beiträgt.

Die Verbindung III verbindet sich mit zahlreichen bekannten Riechstoffingredientien natürlichen oder synthetischen Ursprungs, wobei die Palette der natürlichen Riechstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,
- Alkohole, wie Farnesol, Geraniol, Linalool, Nerol, Phenyläthylalkohol, Rhodinol, Zimtalkohol, Sandalore (3-Methyl-5-(2,2,3-trimethylcyclopent -3-en-1-yl)pentan-2-ol), Sandela (3-Isocamphyl-(5)-cyclohexanol),
- Aldehyde, wie Citral, Helional®, α-Hexylzimtaldehyd, Hydroxycitronellal, Lilial® (p-tert.Butyl-α-methyldihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyläthoxolat (Citronellyl . O - CO -CO . $OC_2H_5$), Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat, 2-Aethyl-6,6-dimethyl (und 2,3,6,6-Tetramethyl)-2-cyclohexen-1-carbonsäureäthylester ("Givescone"), Rosacetol (Trichlormethylbenzylacetat), Vetynal (acetyliertes Caryophyllen),
- Lactone, wie γ-Undecalacton,
- verschiedene, in der Parfümerie oft benützte Komponenten, wie Ketonmoschus, Indol, p-Menthan-8-thiol-3-on, Methyleugenol.

Bemerkenswert ist ferner die Art und Weise, wie die Verbindung III die Geruchsnoten einer breiten Palette bekannter Kompositionen abrundet und harmonisiert, ohne aber in unangenehmer Weise zu dominieren. Genannt seien in diesem Zusammenhang: Kompositionen mit blumigen, z.B. Jasmin- oder Rosennoten, aber auch holzige, Chypre-, animalische, tabakartige und Patchouli-Kompositionen, etc.

Die Verbindung der Formel III lässt sich in weiten Grenzen einsetzen, die beispielsweise von ca. 0,1 (Detergentien) - ca. 5% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 0,1% und ca. 3%. Die mit III hergestellten Kompositionen lassen sich für alle Arten von parfümierten Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorantien, Detergentien, Tabak, etc.).

Die Verbindung III kann demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Beispiel 1

26,45 g (150 mMol) Verbindung IV (89%ig) werden in 250 ml THF gelöst und mit einer Lösung von 47,63 g (350 mMol) $KH_2PO_4$ in 350 ml Wasser versetzt. Die weisse Suspension wird im Eisbad abgekühlt und es werden danach innert einer Stunde bei 4-8°C 106 ml (155 mMol) NaOCl-Lösung (11%ig) zugetropft, dann bei ~5°C nochmals 10 ml (15 mMol) der NaOCl-Lösung, und nach insgesamt 2,5 Stunden wird aufgearbeitet. Das Reaktionsgemisch wird viermal mit je 100 ml Aether extrahiert, die gesamte organische Phase mit 100 ml Wasser gewaschen und diese Wasserphase nochmals mit 50 ml Aether extrahiert. Die organische Phase wird über $MgSO_4$ getrocknet, abfiltriert und am Rotavapor eingedampft. Es ergeben sich 36,19 g einer hellgelben Flüssigkeit von IIa + IIb, welche ungereinigt weiterverarbeitet werden. Nach Reinigung durch Niederdruck-säulenchromatographie an Silicagel zeigt das Hauptprodukt IIa folgende physikalischen Daten:

IR (Film): 3350, 1450, 1385, 1365, 1223, 1190, 1160, 1150, 1120, 1030, 990, 902, 857, 706, 635 $cm^{-1}$. H-NMR (400 MHz, $CDCl_3$, Entkopplung, NOE) [nuclear Overhouser effect]: 5,77 ppm (1H, enges Triplett); 4,68 ppm (1H, schmales Multiplett); 2,33 ppm (1H, aufgespaltenes Doppeldublett); 1,295 und 1,03 und 0,782 ppm (je 3H Singulette für anguläre Methylgruppen).

Beispiel 2

60,85 g (210,6 mMol) Redal [= $NaAl(OCH_2CH_2OCH_3)_2H_2$)]

(70%ig) werden in 190 ml Toluol vorgelegt und es werden 24,10 g (105,3 mMol) Verbindung II (Rohprodukt) aus 70%igem IV in 190 ml Toluol während 80 Minuten bei 23-33°C zugetropft. Man rührt die leicht trübe Mischung 3,5 Stunden bei 55°C weiter, hydrolysiert im Eisbad mit Wasser und arbeitet wie folgt auf: Die organische Phase wird viermal mit je 80 ml Wasser gewaschen, die gesamte Wasserphase dreimal mit je 70 ml Hexan extrahiert und die Hexanphase mit Wasser neutral gewaschen. Die vereinigten organischen Phasen werden über $MgSO_4$ getrocknet und eingedampft. Es ergeben sich 19,58 g einer hellgelben Flüssigkeit. Das Rohprodukt wird mit 35 ml 5N KOH in 200 ml Isopropanol während 2,5 Stunden bei Rückflusstemperatur gehalten. Danach wird mit 100 ml Wasser und 100 ml Hexan verdünnt, die Phasen werden getrennt, die Wasserphase wird dreimal mit je 100 ml Hexan extrahiert, die vereinigten organischen Phasen werden mit Wasser neutral gewaschen und das Waschwasser wird nochmals mit Hexan extrahiert. Die organische Phase wird über $MgSO_4$ getrocknet, abfiltriert und eingedampft. Es resultieren 16,37 g einer gelben Flüssigkeit (54% α-Ambrinol). Das Rohprodukt wird durch Kugelrohrdestillation von Kopffraktion und Rückstand getrennt (0,08 Torr/60-80°). Es resultieren 9,33 g leicht gelbliche Flüssigkeit mit dem typisch starken animalisch, erdig-camphrig und moschusartigen α-Ambrinolgeruch (Gehalt an α-Ambrinol 58%; Gehalt an epi-α-Ambrinol:17%. Die Anreicherung des α-Ambrinols kann durch fraktionierte Destillation oder durch Chromatographie erfolgen.

Beispiel 3

In einer mit Stickstoff vorgespülten Apparatur werden 5,95 g (156,9 mMol) Lithiumaluminiumhydrid in 250 ml Aether vorgelegt. 35,90 g (156,9 mMol) Verbindung II (Rohprodukt) werden in 250 ml Aether innert 1 Stunde bei 24-30°C zugetropft. Es wird mit 30 ml Aether nachgespült und die graue Suspension wird 2 Stunden bei Rückflusstemperatur gehalten. Danach wird mit 200 ml gesättigter Pottasche-Lösung hydrolysiert, dann wie folgt aufgearbeitet: Die organische Phase wird dreimal mit je 50 ml Wasser gewaschen, die vereinten Wasserphasen viermal mit je 100 ml Aether extrahiert und diese Aetherphase viermal mit je 50 ml Wasser neutral gewaschen. Die Aetherphasen werden vereint, über $MgSO_4$ getrocknet, es wird abfiltriert und eingedampft. Es resultieren 28,52 g einer gelben Flüssigkeit. Das Rohprodukt α-Ambrinol wird mit 100 ml 2N KOH in Aethanol während total 4 Stunden bei Rückflusstemperatur gehalten. Die braune Lösung wird danach mit 100 ml Wasser verdünnt, achtmal mit je 50 ml Hexan extrahiert, die organische Phase mit Wasser neutral gewaschen, über $MgSO_4$ getrocknet, abfiltriert und eingedampft. Das Rohprodukt (19,67 g) wird nochmals 2 Stunden mit KOH/EtOH bei Rückflusstemperatur gehalten, aufgearbeitet und über eine 2 cm Vigreux-Kolonne bei 0,08 Torr fraktioniert. Das anfallende α-Ambrinol ist 50%ig.

Beispiel 4

9,57 g (219,1 mMol) Natriumhydrid (55%ig) werden in 360 ml THF vorgelegt und 41,76 g (182,6 mMol) Verbindung II (Rohprodukt) in 360 ml THF während 1,5 Stunden bei 21-25°C zugetropft. Die graue Suspension wird senfgelb. Es wird 4 Stunden bei 40°C weitergerührt, mit gesättigter Pottasche-Lösung im Eisbad hydrolysiert und aufgearbeitet. Die Wasserphase wird viermal mit je 120 ml Aether extrahiert, die gesamte organische Phase viermal mit je 100 ml Wasser gewaschen und die Wasserphase zweimal mit je 100 ml Aether extrahiert. Die vereinigten organischen Phasen werden über $MgSO_4$ getrocknet, abfiltriert und eingedampft. Es verbleiben 31,79 g einer Flüssigkeit, welche mit 50 ml 5N KOH in 300 ml Isopropanol während 2 1/2 Stunden bei Rückflusstemperatur gehalten wird. Danach wird mit Wasser verdünnt und mit Hexan extrahiert. Die organische Phase wird mit Wasser neutralgewaschen, über $MgSO_4$ getrocknet, abfiltriert und eingedampft. Es resultieren 27,67 g dunkelgelbe Flüssigkeit, welche durch Kugelrohrdestillation bei 0,08 Torr und 60-80°C vom Rückstand getrennt werden. Ausbeute: 18,12 g (66% α- und 12,5% β-Epoxyd). Eine durch Chromatographie an Silicagel gereinigte Probe zeigt folgende physikalischen Daten für III:

GC: 90-91%, 72% α-Epoxyd 18% β-Epoxyd).

IR (Film): 1660, 1450, 1382, 1360, 1298, 1288, 1217, 1191 1018, 1005, 948, 925, 872, 840/830/820, 775, 745, 692, 660 $cm^{-1}$. H-NMR (400 MHz, $CDCl_3$): (für Hauptprodukt= α-Epoxyd): 5,865 ppm (1H, schmales Multiplett); 3,163 ppm (1H, Singulett), 1,352 ppm (3H, Singulett); 0,928 ppm (3H, Singulett); 0,748 ppm (3H, Singulett). (Nebenprodukt= β-Epoxyd): 5,92 ppm (1H, schmales Multiplett); 3,185 ppm (1H, Singulett); 1,352 ppm (3H, Singulett); 0,928 ppm (3H, Singulett); 0,71 ppm (3H, Singulett). In $C_6D_6$ sind die drei Singulette für die je drei angulären Methylgruppen getrennt ersichtlich.

MS: Hauptprodukt: $M^+$ = 192(12); m/e = 177(5), 176(7), 163(3), 161(9), 159(4), 150(37), 135(20), 121(17), 107(44), 94(100), 79(39), 67(15), 55(21), 43(64). Nebenprodukt: $M^+$ = 192(13); m/e = 177(5), 176(3), 163(3),

161(3), 159(4), 150(30), 135(18), 121(19), 107(47), 94(100), 79(48), 67(18), 55(22), 43(72).
Geruch: Fäkalisch, animalisch, Schweiss der Wildkatze.

Beispiel 5

0,10 g (2,6 mMol) Lithiumaluminiumhydrid werden in 10 ml Aether vorgelegt und 0,50 g (2,6 mMol) Verbindung III in 10 ml Aether zugegeben. Danach wird 1,5 Stunden bei Rückfluss gehalten, im Eisbad mit gesättigter Pottasche-Lösung hydrolysiert und aufgearbeitet. Die organische Phase wird mit Wasser gewaschen und die gesamte Wasserphase mit Aether extrahiert. Die vereinigten organischen Phasen werden über $MgSO_4$ getrocknet, abfiltriert und am Rotavapor eingedampft. Es resultieren 0,53 g einer farblosen Flüssigkeit mit dem starken camphrig-indolig-animalischen Geruch von α-Ambrinol. Gemäss Gaschromatogramm enthält das Produkt 76% α-Ambrinol und 13% epi-α-Ambrinol.

Beispiel 6

Kompositionen

a) Patchoulinote

| | Gewichtsteile | |
|---|---|---|
| α-Hexylzimtaldehyd | 30 | |
| Vanillin | 10 | |
| Sandela | 10 | |
| Lilial | 40 | |
| Indol (2,3-Benzpyrrol) 10% in DPG | 2 | |
| Coumarin | 25 | |
| Fixolide | 5 | |
| Isoeugenol | 7 | |
| Pêche pure (γ-Undecalacton) | 2 | |
| Vetynal (acetyliertes Caryophyllen) | 30 | |
| Eugenol | 50 | |
| Cannelier feuilles ess. rest. (Zimtblätteröl) | 15 | |
| Patchouli-Essenz | 90 | |
| Hydroxycitronellal | 40 | |
| ß-Phenyläthylalkohol | 100 | |
| Geraniol | 30 | |
| Benzylacetat | 35 | |
| Linalylacetat | 40 | |
| Omega-n-Undecylenaldehyd | 3 | |
| Linalool | 30 | |
| DPG | 406 | 401 |
| Verbindung III | | 5 |
| | 1000 | 1000 |

Durch den Zusatz an Verbindung III gewinnt die ursprüngliche Komposition an Volumen und Kraft. Die animalische und die Patchoulinote werden verstärkt.

b) Rosennote

| | Gewichtsteile | |
|---|---|---|
| Geraniol | 120 | |
| Citronellol | 60 | |
| Rhodinol | 100 | |
| Phenyläthylalkohol | 580 | |
| Linalool | 20 | |
| Rosacetol (Trichlormethylbenzylacetat) | 50 | |
| Acetate DMBC (Dimethylbenzylcarbinylacetat) | 30 | |
| DPG | 40 | 37 |
| Verbindung III | | 3 |
| | 1000 | 1000 |

Durch den Zusatz an Verbindung III gewinnt die ursprüngliche Komposition an Volumen und Kraft, und es tritt eine angenehme Verstärkung und Fixierung der Holznote auf. Gleichzeitig gewinnt die Komposition an Dichte.

## Patentansprüche

### Patentansprüche für folgende Vertragsstaaten : CH, DE, FR, GB, IT, LI, NL

1. Verfahren zur Herstellung von 2-Hydroxy-2,5,5-trimethyl -1,2,3,4,4a,5,6,7-octahydronaphthalin, dadurch gekennzeichnet, dass man ein 8-(Chlor oder Brom, insbesondere Chlor)-2-hydroxy-2,5,5-trimethyl-2,3,4,4a, 5,6,7,8 -octahydronaphthalin, gegebenenfalls im Gemisch mit einem 1-(Chlor oder Brom, insbesondere Chlor)-2-hydroxy-2,5,5-trimethyl-1,2,3,4,4a,5,6,7 -octahydronaphthalin, bzw. das 1,2-Epoxyd davon, reduziert.

2. Verfahren nach Anspruch 1, dadurch gekennzeichnet, dass man mit einem komplexen Hydrid, insbesondere mit $LiAlH_4$ oder einem Derivat von $NaAlH_4$ reduziert.

3. 1,2-Epoxy-2,5,5-trimethyl-1,2,3,4,4a,5,6,7-octahydronaphthalin.

4. 1-(Chlor oder Brom, insbesondere Chlor)-2-hydroxy-2,5,5-trimethyl-1,2,3,4,4a,5,6,7-octahydronaphthalin.

5. 8-(Chlor oder Brom, insbesondere Chlor)-2-hydroxy-2,5,5-trimethyl-2,3,4,4a,5,6,7,8-octahydronaphthalin.

6. Riechstoffkomposition, gekennzeichnet durch einen Gehalt an 1,2-Epoxy-2,5,5-trimethyl-1,2,3,4,4a,5,6, 7 -octahydronaphthalin.

7. Verfahren zur Herstellung von Riechstoffkompositionen, dadurch gekennzeichnet, dass man 1,2-Epoxy-2,5,5-trimethyl-1,2,3,4,4a,5,6,7 -octahydronaphthalin mit den üblichen Formulierungshilfsstoffen und gegebenenfalls mit anderen Riechstoffen zusammenbringt.

8. Verwendung von 1,2-Epoxy-2,5,5-trimethyl-1,2,3,4,4a,5,6,7 -octahydronaphthalin als Riechstoff.

**Patentansprüche für folgenden Vertragsstaat : ES**

1. Verfahren zur Herstellung von 2-Hydroxy-2,5,5-trimethyl -1,2,3,4,4a,5,6,7-octahydronaphthalin, dadurch gekennzeichnet, dass man ein 8-(Chlor oder Brom, insbesondere Chlor)-2-hydroxy-2,5,5-trimethyl-2,3,4,4a, 5,6,7,8 -octahydronaphthalin, gegebenenfalls im Gemisch mit einem 1-(Chlor oder Brom, insbesondere Chlor)-2-hydroxy-2,5,5-trimethyl-1,2,3,4,4a,5,6,7 -octahydronaphthalin, bzw. das 1,2-Epoxyd davon, reduziert.

2. Verfahren nach Anspruch 1, dadurch gekennzeichnet, dass man mit einem komplexen Hydrid, insbesondere mit $LiAlH_4$ oder einem Derivat von $NaAlH_4$ reduziert.

3. Riechstoffkomposition, gekennzeichnet durch einen Gehalt an 1,2-Epoxy-2,5,5-trimethyl-1,2,3,4,4a,5,6, 7 -octahydronaphthalin.

4. Verfahren zur Herstellung von Riechstoffkompositionen, dadurch gekennzeichnet, dass man 1,2-Epoxy-2,5,5-trimethyl-1,2,3,4,4a,5,6,7 -octahydronaphthalin mit den üblichen Formulierungshilfsstoffen und gegebenenfalls mit andern Riechstoffen zusammenbringt.

5. Verwendung von 1,2-Epoxy-2,5,5-trimethyl-1,2,3,4,4a,5,6,7 -octahydronaphthalin als Riechstoff.

**Claims**

**Claims for the following Contracting States : CH, DE, FR, GB, IT, LI, NL**

1. A process for the manufacture of 2-hydroxy-2,5,5-trimethyl -1,2,3,4,4a,5,6,7-octahydronaphthalene, characterized by reducing an 8-(chloro or bromo, especially chloro)-2-hydroxy-2,5,5-trimethyl-2,3,4,4a,5,6,7,8-octahydronaphthalene, optionally in admixture with a 1 -(chloro or bromo especially chloro)-2-hydroxy -2,5,5-trimethyl-1,2,3,4,4a,5,6,7-octahydronaphthalene, or the 1,2-epoxide thereof.

2. A process according to claim 1, characterized in that the reduction is carried out with a complex hydride, especially with $LiAlH_4$ or a derivative of $NaAlH_4$.

3. 1,2-Epoxy-2,5,5-trimethyl-1,2,3,4,4a,5,6,7-octahydronaphthalene.

4. 1 -(Chloro or bromo, especially chloro)-2-hydroxy-2,5,5-trimethyl-1,2,3,4,4a,5,6,7-octahydronaphthalene.

5. 8-(Chloro or bromo, especially chloro)-2-hydroxy-2,5,5-trimethyl-1,2,3,4,4a,5,6,7-octahydronaphthalene.

6. An odorant composition, characterized by a content of 1,2-epoxy-2,5,5-trimethyl-1,2,3,4,4a,5,6,7-octahydronaphthalene.

7. A process for the manufacture of odorant compositions, characterized by bringing 1,2-epoxy-2,5,5-trimethyl-1,2,3,4,4a,5,6,7-octahydronaphthalene together with the usual formulation adjuvants and, if desired, with other odorants.

8. The use of 1,2-epoxy-2,5,5-trimethyl-1,2,3,4,4a, 5,6,7-octahydronaphthalene as an odorant.

**Claims for the following Contracting State : ES**

1. A process for the manufacture of 2-hydroxy-2,5,5-trimethyl -1,2,3,4,4a,5,6,7-octahydronaphthalene, characterized by reducing an 8-(chloro or bromo, especially chloro)-2-hydroxy-2,5,5-trimethyl-1,2,3,4,4a,5,6,7-octahydronaphthalene, optionally in admixture with a 1-(chloro or bromo especially chloro)-2-hydroxy -2,5,5-trimethyl-1,2,3,4,4a,5,6,7-octahydronaphthalene, or the 1,2-epoxide thereof.

2. A process according to claim 1, characterized in that the reduction is carried out with a complex hydride, especially with $LiAlH_4$ or a derivative of $NaAlH_4$.

3. An odorant composition, characterized by a content of 1,2-epoxy-2,5,5-trimethyl-1,2,3,4,4a,5,6,7-octahydronaphthalene.

4. A process for the manufacture of odorant compositions, characterized by bringing 1,2-epoxy-2,5,5-trimethyl-1,2,3,4,4a,5,6,7-octahydronaphthalene together with the usual formulation adjuvants and, if desired, with other odorants.

5. The use of 1,2-epoxy-2,5,5-trimethyl-1,2,3,4,4a, 5,6,7-octahydronaphthalene as an odorant.

## Revendications

### Revendications pour les Etats contractants suivants : CH, DE, FR, GB, IT, LI, NL

1. Procédé de préparation du 2-hydroxy-2,5,5-triméthyl-1,2,3,4,4a,5,6,7-octahydronaphtalène, caractérisé en ce que l'on réduit un 8-(chloro ou bromo, plus spécialement chloro)-2-hydroxy-2,5,5-triméthyl-2,3,4,4a, 5,6,7,8-octa-hydronaphtalène, éventuellement en mélange avec un 1-( chloro ou bromo, plus spécialement chloro)-2-hydroxy-2, 5,5-triméthyl-1,2,3,4,4a,5,6,7-octanydronaphtalène ou son 1,2-époxyde.

2. Procédé selon la revendication 1, caractérisé en ce que l'on réduit à l'aide d'un hydrure complexe, en particulier $LiAlH_4$ ou un dérivé de $NaAlH_4$.

3. Le 1,2-époxy-2,5,5-triméthyl-1,2,3,4,4a,5,6,7-octahydronaphtalène.

4. 1-(chloro ou bromo, plus spécialement chloro)-2-hydroxy-2,5,5-triméthyl-1,2,3,4,4a,5,6,7-octahydronaphtalène.

5. 8-(chloro ou bromo, plus spécialement chloro)-2-hydroxy-2,5,5-triméthyl-2,3,4,4a,5,6,7,8-octahydronaphtalène.

6. Composition odorante caractérisée en ce qu'elle contient du 1,2-époxy-2,5,5-triméthyl-1,2,3,4,4a,5,6,7-octahydronaphtalène.

7. Procédé de préparation de compositions odorantes, caractérisé en ce que l'on mélange le 1,2-époxy-2,5,5-triméthyl -1,2,3,4,4a,5,6,7-octahydronaphtalène avec les produits auxiliaires de formulation usuels et le cas échéant d'autres matières odorantes.

8. Utilisation du 1,2-époxy-2,5,5-triméthyl-1,2,3,4,4a, 5,6,7-octahydronaphtalène en tant que matière odorante.

### Revendications pour l'Etat contractant suivant : ES

1. Procédé de préparation du 2-hydroxy-2,5,5-triméthyl-1 ,2,3,4,4a,5,6,7-octahydronaphtalène, caractérisé en ce que l'on réduit un 8-(chloro ou bromo, plus spécialement chloro)-2-hydroxy-2,5,5-triméthyl-2,3,4,4a, 5,6,7,8-octa-hydronaphtalène, éventuellement en mélange avec un 1-(chloro ou bromo, plus spécialement chloro)-2-hydroxy-2,5,5-triméthyl-1,2,3,4,4a,5,6,7-octahydronaphtalène ou son 1,2-époxyde.

2. Procédé selon la revendication 1, caractérisé en ce que l'on réduit à l'aide d'un hydrure complexe, en particulier à l'aide de $LiAlH_4$ ou d'un dérivé de $NaAlH_4$.

3. Composition odorante , caractérisée en ce qu'elle contient du 1,2-époxy-2,5,5-triméthyl-1,2,3,4,4a,5,6,7-octahydronaphtalène.

4. Procédé de préparation de compositions odorantes, caractérisé en ce que l'on mélange du 1,2-époxy-2,5,5-triméthyl -1,2,3,4,4a,5,6,7-octahydronaphtalène avec les produits auxiliaires de formulation usuels et le cas échéant d'autres matières odorantes.

5. Utilisation du 1,2-époxy-2,5,5-triméthyl-1,2,3,4,4a, 5,6,7-octahydronaphtalène en tant que matière odorante.